# EUROPEAN PATENT APPLICATION

(11) **EP 2 544 007 A1**
(43) Date of publication of application: **09.01.2013**
(21) Application number: 11173081.8
(22) Date of filing: 07.07.2011
(51) Int. Cl.: G01N 33/68

(54) **Biomarker of renal impairment**

(71) Applicant: Atlas Antibodies AB, 106 91 Stockholm (SE)
(72) Inventor: Schwenk, Jochen, 118 53 Stockholm (SE); Nilsson, Peter, 163 52 Spånga (SE); Uhlén, Mathias, 182 79 Stocksund (SE)
(74) Representative: Wirén, Anders

(57) **Abstract**

There is provided a method of determining whether a subject belongs to a first or a second group of subjects, wherein the risk of having or developing of a renal impairment is higher in the first group than in the second group, comprising the steps of:
a) measuring an amount of fibulin 1 in a sample from the subject to obtain a sample value;
b) comparing the sample value to a reference value; and
if the sample value is higher than the reference value,
c1) concluding that the subject belongs to the first group; and
if the sample value is lower than the previous sample value,
c2) concluding that the subject belongs to the second group.
Associated means are also provided.

## Description

### Technical field

The present disclosure relates to the field of diagnosis or monitoring of a renal condition.

### Background

### Kidney toxicity

The human kidney has approximately 1 million functional units, nephrons, that filter about 150-180 liters of plasma per day. These nephrons regulate fluid, electrolyte, and acid-base balance as well as eliminate waste products. In the filtering process, the components of the filtrate may become significantly concentrated, as high as >100-fold their original concentration, leading to increased intracellular concentrations. The kidney is therefore a sensitive organ when it comes to toxic substances, since their renal accumulation may be substantially higher than in the rest of the body.

The assessment of renal damage is important in many different aspects. Renal damage may occur from chronic kidney disease as well as from acute kidney injury (AKI). Chronic kidney disease develop slowly over time and may have a number of causes that may be either aquired or congenital. Examples of aquired chronic kidney diseases are: Diabetic nephropathy, the leading cause of kidney disease in the USA; Glomerulonephritis, inflammation and damage of the filtration system of the kidneys, caused by e.g. postinfectious conditions and lupus; and analgesic nephropathy, caused by use of analgesics over long durations of time. Examples of congenital chronic kidney diseases are: polycystic kidney disease, where both kidneys have multiple cysts; obstructive uropathy, where urine flow is blocked and the kidney function therefore impaired; and renal dysplasia, a deformation of the kidneys.

Acute kidney injury (AKI) involves a rapid loss of kidney function and may be caused by intake of chemicals such as contrast agents, antibiotics, and chemotherapeutics. It is thus of utmost importance during drug development to monitor patients involved in clinical trials for AKI. The Kidney Disease: Improving Global Outcomes (KDIGO) foundation has recommended the following definition of AKI: Serum creatinine (SCr) rises by ≥ 26µmol/L within 48 hours or SCr rises ≥ 1.5 fold from the reference value, which is known or presumed to have occurred within one week or urine output is < 0.5m1/kg/hr for > 6 consecutive hours. Staging of AKI may be based on either serum creatinine (SCr) level increase or urine output criteria. The KDIGO foundation has further recommended the following staging of AKI: Stage 1: SCr increase ≥ 26 µmol/L within 48 h or increase ≥ 1.5 to 1.9 times reference SCr or urine output < 0.5 mL/kg/h for > 6 consecutive hours; Stage 2: Increase ≥ 2 to 2.9 times reference SCr or urine output < 0.5 mL/kg/h for > 12 hours; Stage 3: SCr increase ≥ 354 µmol/L or increase ≥ 3 times reference SCr or commenced on renal replacement therapy (RRT) or urine output < 0.3 mL/kg/h for > 24 hours or anuria for 12 hours.

The kidney is a major site for drug-induced toxicity and monitoring its functionality for early onset of AKI is desireable in order to withdraw any toxic medication and start adequate treatment as early as possible. Treatment of AKI may include appropriate fluid therapy, such as a sodium chloride solution (for prevention and early stage disease), or renal replacement therapy (in advanced disease).

There is a need for reliable and sensitive biomarkers for renal impairments to detect early signs of kidney toxicity and to monitor progression of disease. Today, measurement of serum creatinine (SCr) and blood urea nitrogen (BUN) are used as indication of renal injury, and, as mentioned above, SCr increase is currently used as a definition of AKI. Both these biomarkers have, however, severe limitations. SCr levels are only increased after substantial renal injury has occurred, particularly in patients with a large renal reserve. BUN is not a reliable marker of renal injury since urea may be reabsorbed by different areas of the nephron, and also, increased levels of BUN may reflect increased urea production rather than renal impairment. Both markers are further influenced by factors such as dehydration and loss of muscle mass, which are common symptoms in kidney patients.

A number of markers, such as kidney injury molecule 1 (KIM-1), neutrophil gelatinase-associated lipocalin (NGAL), cystatin C, fatty acid binding protein-liver type (L-FABP), alpha-GST, interleukin-18 (IL-18), and osteopontin, have been proposed to increase sensitivity of kidney injury detection, and may optionally be used for additional information in clinical trials, but none of these markers are in routine use in clinics today. New and improved markers would be of interest both for detection and montoring of the different types of renal impairment.

### Summary

It is an object of some aspects of the present disclosure to provide means and methods for the diagnosis, characterization or monitoring of a renal condition. An object of some embodiments of these aspects is to provide such means and methods for a subject participating in a clinical trial.

The following is a non-limiting and itemized listing of embodiments of the present disclosure, presented for the purpose of providing various features and combinations provided by the invention in certain of its aspects.

### ITEMS

1. A method of determining whether a subject belongs to a first or a second group of subjects, wherein the risk of having or developing of a renal impairment is higher in the first group than in the second group, comprising the steps of:
   a) measuring an amount of fibulin 1 in a sample from the subject to obtain a sample value;
   b) comparing the sample value to a reference value; and if the sample value is higher than the reference value,
   c1) concluding that the subject belongs to the first group; and
      if the sample value is lower than the previous sample value,
   c2) concluding that the subject belongs to the second group.
2. A method according to item 1, wherein the renal impairment is selected from glomerulonephritis, diabetic nephropathy and obstructive uropathy.
3. A method according to item 1, wherein the subject undergoes a clinical trial of a treatment.
4. A method according to any one of items 1-3, wherein the reference value is a previous sample value obtained from a previous sample taken earlier than the sample of step a).
5. A method of monitoring a renal condition in a subject having a renal disorder, comprising the steps of:
   a) measuring an amount of fibulin 1 in a sample from the subject to obtain a sample value;
   b) comparing the sample value to a previous sample value obtained from an earlier sample taken from the subject; and
      if the sample value is higher than the previous sample value,
   c1) concluding that the renal condition has worsened since the earlier sample was taken; and/or
      if the sample value is lower than the previous sample value,
   c2) concluding that the renal condition has improved since the earlier sample was taken.
6. A method according to item 5, wherein the subject participates in a clinical trial of a potential treatment of the renal disorder and the earlier sample was taken earlier in the clinical trial process or before the clinical trial was commenced.
7. A method according to item 5 or 6, wherein the renal disorder is selected from glomerulonephritis, diabetic nephropathy and obstructive uropathy.
8. A method of disqualifying a subject from a clinical trial of a treatment, comprising the steps of:
   a) measuring an amount of fibulin 1 in a sample from the subject to obtain a sample value;
   b) comparing the sample value to a reference value; and
      if the sample value is higher than the reference value,
   c) disqualifying the subject from the clinical trial.
9. A method according to any one of item 3, 4 and 6-8, wherein the treatment comprises an application of a drug to the subject.
10. A method of treatment of a subject, comprising the steps of:
   a) measuring an amount of fibulin 1 in a sample from the subject to obtain a sample value;
   b) comparing the sample value to a reference value; and
      if the sample value is higher than the reference value,
   c) treating the subject with a renal disorder treatment regimen.
11. A method according to any one of the preceding items, wherein the sample is an optionally modified sample derived from urine or blood, such as an optionally diluted serum or plasma sample.
12. A method according to any one of the preceding items, wherein step a) comprises contacting the sample with an affinity ligand capable of selective interaction with fibulin 1.
13. A method according to item 12, wherein the affinity ligand is capable of selective interaction with a peptide whose amino acid sequence consists of SEQ ID NO:1 or SEQ ID NO:2.
14. A method according to item 12 or 13, further comprising quantifying the interaction between the affinity ligand and fibulin 1 to obtain the sample value.
15. Method according to any one of the preceding items, wherein step a) comprises a sandwich assay.
16. Method according to item 15, wherein the sandwich assay is an ELISA sandwich assay.
17. A kit comprising:
   a) a quantifiable affinity ligand capable of selective interaction with a fibulin 1 protein;
   b) reagents necessary for quantifying the amount of the quantifiable affinity ligand of a);
   a') a quantifiable affinity ligand capable of selective interaction with Serum Creatinine, KIM-1, Cystatin C, L-FABP, alpha-GST, pi-GST, Urinary Collagen IV, Alpha-1-Microglobulin, Beta-2-Microglobulin, Calbindin, Clusterin, Connective Tissue Growth Factor, Glutathione S-Transferase alpha, Urinary albumin, Microalbumin, NGAL, Osteopontin, Tamm-Horsfall Urinary Glycoprotein, Tissue Inhibitor of Metalloproteinases 1, TFF3, VEGF, GC, SLC13A3, GDA, MACF1, MAPK3, RBP4, SFXN1 or BBOX1; and
   b') reagents necessary for quantifying the amount of the quantifiable affinity ligand of a'),
      wherein the reagents of b) and b') may be the same or different.
18. A product comprising an affinity ligand capable of selective interaction with fibulin 1, the affinity ligand being immobilized onto a solid support.
19. A product according to item 18, wherein the solid support is a plate for a sandwich assay or a bead for a bead-based assay.
20. Kit comprising:
   a) a first affinity ligand capable of selective interaction with a fibulin 1 protein;
   b) a quantifiable second affinity ligand capable of interaction with the fibulin 1 protein when bound to the first affinity ligand; and optionally
   c) a solid support onto which the first affinity ligand is immobilized or which is prepared for immobilization of the first affinity ligand.
21. Kit according to item 20, wherein the quantifiable second affinity ligand is capable of selective interaction with the fibulin 1 protein when bound to the first affinity ligand.
22. Kit according to item 20 or 21, wherein the first affinity ligand interacts with a first fibulin 1 amino acid sequence and the quantifiable second affinity ligand interacts with a second fibulin 1 amino acid sequence, which is different from the first first amino acid sequence.
23. Kit according to any one of items 20-22, wherein the first fibulin 1 amino acid sequence is SEQ ID NO:1 or SEQ ID NO:2.
24. Kit according to any one of items 20-23, wherein the second fibulin 1 amino acid sequence is SEQ ID NO:1 or SEQ ID NO:2.
25. Kit according to any one of items 20-24, wherein the quantifiable second affinity ligand is labeled with a quantifiable label.
26. Kit according to any one of items 20-25, further comprising an agent capable of binding the quantifiable second affinity ligand, the agent being labeled with a quantifiable label.
27. Kit according to item 26, wherein the quantifiable second affinity ligand is an antibody and the agent is an antibody capable of binding the Fc region of the quantifiable second affinity ligand.
28. Kit according to any one of items 25-27, wherein the quantifiable label is selected from the group consisting of fluorescent dyes and metals, chromophoric dyes, chemiluminescent compounds and bioluminescent proteins, enzymes, radioisotopes, particles and quantum dots.
29. Kit according to item 28, in which the quantifiable label is an enzyme, further comprising a chromogenic ELISA substrate.
30. Kit according to item 28 or 29, wherein the quantifiable label is an enzyme selected from horseradish peroxidase, alkaline phaosphatase, beta-D-galactosidase and beta-lactamase.
31. Kit according to anyone of items 20-30, wherein the first affinity ligand is an antibody.
32. Kit according to anyone of items 20-31, wherein the quantifiable second affinity ligand is an antibody.
33. Kit according to anyone of items 20-32, wherein the solid support comprises wells in which the first affinity ligand is immobilized or which are prepared for immobilization of the first affinity ligand.
34. Use *ex vivo* of fibulin 1 as a diagnostic marker for a renal impairment.
35. Use *ex vivo* of fibulin 1 as a diagnostic marker for a renal impairment in a clinical trial of a treatment.
36. Use *ex vivo* of an affinity ligand capable of selective interaction with fibulin 1 as a diagnostic agent for a renal impairment.
37. Use *ex vivo* of an affinity ligand capable of selective interaction with fibulin 1 as a diagnostic agent for a renal impairment in a clinical trial of a treatment.
38. Use according to item 36 or 37, wherein the affinity ligand is immobilized onto a solid support.

### Brief description of the figures

Figure 1 shows a schematic alignment of the four reported isoforms of fibulin 1. Isoform A is a shorter splice variant, and isoform B and C have alternative sequences in the C-terminal part compared to the canonical isoform D. Epitope regions for the polyclonal antibodies denoted HPA 1-3, as well as the monoclonal antibody sc-25281 from Santa Cruz Biotechnology are also shown.
Figure 2 shows a boxplot of differences in fibulin 1 protein concentration in plasma between patients diagnosed with glomerulonephritis (grey boxes) and healthy controls (white boxes).
Figure 3 shows discriminatory capacity of various proteins in distinguishing between glomerulonephritis patients and healthy controls. Solid black circles represent the detection of fibulin 1 by different antibodies. Non-filled circles represent the detection of other proteins.

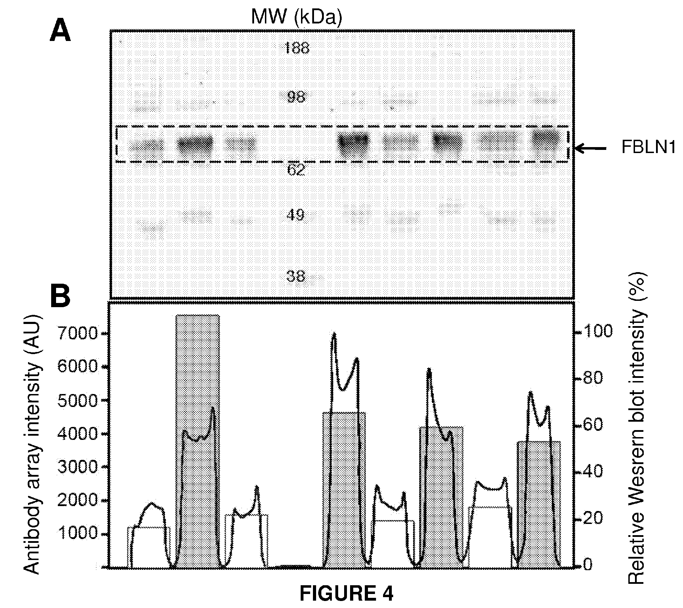

Figure 4A shows Western blot validation of the HPA1 antibody targeting fibulin 1. Lanes 1, 3, 6, and 8 show samples from healthy individuals, lanes 2, 5, 7, and 9 show samples from patients diagnosed with glomerulonephritis, the marker size is indicated in lane 4. Figure 4B shows the overlap of the antibody array intensity as determined in Example 4 (bars), and the signals from the Western blot in Figure 4A translated into relative intensities (line chart). Signals from Western blot and antibody arrays are shown for representative patients diagnosed with glomerulonephritis (GN, grey bars) and matched controls (NL, white bars).
Figure5 shows a boxplot of differences in fibulin 1 protein concentration in plasma from an independent cohort glomerulonephritis patients between patients diagnosed with glomerulonephritis (grey boxes) and healthy controls (white boxes).
Figure 6 shows a boxplot of differences in fibulin 1 protein concentration in plasma between patients diagnosed with four different renal disorders (grey boxes) and their respective matched controls (white boxes).
   DN represents diabetic nephropathy, OU represents obstructive uropathy, AA represents analgesic abuse and GN represents glomerulonephritis.
Figure 7 shows the results from a sandwich detection assay measuring the levels of the fibulin 1 protein in plasma using four different antibodies as capture agent, and the monoclonal antibody sc-25281 from Santa Cruz Biotechnology (mAb) for detection. Grey boxes represent glomerulonephritis patients and white boxes represent healthy individuals.
Figure 8 shows a boxplot of differences in fibulin 1 protein concentration in urine between patients diagnosed with glomerulonephritis (grey boxes) and healthy controls (white boxes).

### Detailed description

The present disclosure is based on the inventors' finding that the protein fibulin 1 (sometimes referred to as FBLN1) is more abundant in biological samples from subjects having an impaired renal function than in samples from control groups of subjects. Accordingly, the inventors have concluded that fibulin 1 is a biomarker for renal impairment.

Fibulin 1 may for example advantageously be included in a panel of biomarkers detected when diagnosing the renal function of a patient. Examples of such panel biomarkers are given below under the fifth aspect.

Thus, as a first aspect of the present disclosure, there is provided a method of determining whether a subject belongs to a first or a second group of subjects, wherein the risk of having or developing of a renal impairment is higher in the first group than in the second group. The method comprises the steps of:
a) measuring an amount of fibulin 1 in a sample from the subject to obtain a sample value;
b) comparing the sample value to a reference value; and if the sample value is higher than the reference value,
c1) concluding that the subject belongs to the first group; and if the sample value is lower than the previous sample value,
c2) concluding that the subject belongs to the second group.

Most of the diagnosis methods available today cannot detect renal impairments in a subject until a large percentage of the renal function is lost. The inventors believe that the method of the first aspect can be used to detect the impairment at an earlier stage, which increases the chances of successful recovery of the subject.

In the methods of the first aspect, it is determined whether a subject belongs to a first or a second group, wherein subjects of the first group generally have a higher risk of having or developing a renal impairment than subjects of the second group. The division of subjects into the two groups is determined by comparing sample values from the subjects with a reference value. The reference value is thus the determinant for the size of the respective groups; the higher the reference value, the fewer the subjects in the first group and the lower the likelihood that a tested subject belongs to the first group.

The first and the second group may for example consist exclusively of subjects having the same or similar characteristics as the tested subject. For example, the groups may consist of subjects having the same or similar age, race, nationality, residence, genetic characteristics, medical status and/or medical history (such as kidney disease history).

In the Examples below, high levels of fibulin 1 are shown to be associated with four different conditions causing renal impairment (i.e. glomerulonephritis, diabetic nephropathy, obstructive uropathy and analgesic abuse). The inventors thus conclude that fibulin 1 is a general biomarker for renal impairment. The renal impairment may for example be associated with an acquired renal disorder. Thus, the method of the first aspect may relate to the determination of whether a subject belongs to a first or a second group of subjects, wherein the risk of having or developing of an acquired renal disorder is higher in the first group than in the second group. The acquired renal disorder may for example be selected from diabetic nephropathy, glomerulonephritis, hydronephrosis, interstitial nephritis, kidney tumors, Wilms tumor, renal cell carcinoma, lupus nephritis, minimal change disease, nephrotic syndrome, pyelonephritis, obstructive uropathy and renal failure, such as acute renal failure/acute renal injury and stage 5 chronic kidney disease.

In one embodiment, the method of the first aspect may be used to detect an analgesic abuse by the subject.

A particularly interesting application of the method of the first aspect is in a clinical trial of a treatment, where it is often important to detect any renal impairment caused by treatment. Thus, in one embodiment, the subject of the method of the first aspect undergoes a clinical trial of a treatment. The treatment may for example involve the application of a new drug, as the risk of the new drug harming the renal function, e.g. the kidneys, must almost always be taken into account.

The method of the first aspect may involve measuring the fibulin 1 levels in samples taken at various points of time. Accordingly, the reference value may be a fibulin 1 level obtained from a previous sample taken from the subject earlier than the sample of step a). For example, the previous sample may have been taken from the subject at least one month earlier, such as at least 6 months earlier, than the sample of step a). In the context of a clinical trial, the reference value may be a fibulin 1 level obtained from a sample taken from the subject earlier in the clinical trial process or before the clinical trial was commenced. Such a reference value may be preferred as the normal fibulin 1 level may vary from one individual to another. Thus, the increase in the fibulin 1 level rather the absolute level may be decisive in the method according to this embodiment. However, in some embodiments, both the increase in the fibulin 1 level and the absolute level may be taken into account when the conclusion regarding the risk of having or developing the renal disorder is drawn.

As a configuration of the first aspect, there is provided a method for determining a subject's risk of having or developing a renal impairment, comprising the steps of:
a) measuring an amount of fibulin 1 in a sample from the subject to obtain a sample value; and
b) correlating the sample value of step a) to the risk of having or developing the renal impairment.

The correlating of step b) refers to any way of associating data to the obtained sample value so as to determine the risk. With the knowledge of the teachings of the present disclosure, the skilled person may perform step b) without undue burden. For example, the sample value of step a) may be compared to such sample values from reference subjects having renal impairments and/or healthy reference subjects.

The various embodiments of the first aspect described above apply to the configuration *mutatis mutandis.*

The inventors finding may also be used for monitoring a renal condition/renal function in a subject already diagnosed with a renal disorder.

Thus, as a second aspect of the present disclosure, there is provided a method of monitoring a renal condition in a subject having a renal disorder. The method comprises the steps of:
a) measuring an amount of fibulin 1 in a sample from the subject to obtain a sample value;
b) comparing the sample value to a previous sample value obtained a sample taken earlier than the sample of a); and
   if the sample value is higher than the previous sample value,
c1) concluding that the renal condition has worsened since the earlier sample was taken; and/or
   if the sample value is lower than the previous sample value,
c2) concluding that the renal condition has improved since the earlier sample was taken.

The earlier sample may for example be taken from the subject at least 2 weeks, at least 1 month, at least 3 months, at least 6 months or at least 12 months earlier than the sample of step a).

The monitoring of the first aspect may be part of a clinical trial. Thus, in an embodiment of the second aspect, the subject participates in a clinical trial of a potential treatment of the renal disorder and the earlier sample was taken earlier in the clinical trial process or before the clinical trial was commenced. The renal disorder may for example be a congenital or acquired renal disorder. Thus the renal disorder may be selected from the following congenital renal disorders: congenital hydronephrosis; congenital obstruction of urinary tract; duplex kidneys/double kidneys, duplicated ureter, horseshoe kidney, polycystic kidney disease (including autosomal dominant polycystic kidney disease and autosomal recessive polycystic kidney disease), renal agenesis, renal dysplasia, unilateral small kidney, multicystic dysplastic kidney and ureteropelvic junction obstruction (UPJO, may also be an acquired disorder). The renal disorder may also be selected from the aquired renal disorders listed in connection with the first aspect.

When qualifying subjects for a clinical trial of a treatment, it may be beneficial to exclude subjects having renal problems as they may be more sensitive to possible side-effects of the treatment. Thus, as a third aspect of the present disclosure, there is provided a method of disqualifying a subject from a clinical trail of a treatment, comprising the steps of:
a) measuring an amount of fibulin 1 in a sample from the subject to obtain a sample value;
b) comparing the sample value to a reference value; and
   if the sample value is higher than the reference value,
c) disqualifying the subject from the clinical trial.

The treatment of the third aspect may for example comprise an application of a drug to the subject.

It follows from the above that subjects having high levels of fibulin 1 may be in need of treatment, The treatment may be a treatment of the actual disease/disorder or a treatment of the symptom, e.g. the renal impairment.

Thus, as a fourth aspect of the present disclosure, there is provided a method of treatment of a subject, comprising the steps of:
a) measuring an amount of fibulin 1 in a sample from the subject to obtain a sample value;
b) comparing the sample value to a reference value; and
   if the sample value is higher than the reference value,
c) treating the subject with a renal disorder treatment regimen.

The method of the fourth aspect may further comprise refraining from treating the subject with the renal disorder treatment regimen if the sample value is lower than the reference value.

The method of treatment may be limited to the decision-making and treatment. Thus, as a configuration of the fourth aspect, there is provided a method of treatment of a subject, comprising the steps of:
α) comparing a sample value corresponding to a level of fibulin 1 in a sample derived from subject with a reference value; and
   if the sample value is higher than the reference value,
β) treating the subject with a renal disorder treatment regimen.

Ways of obtaining a sample value corresponding to a level of fibulin 1 in a sample are described in the present disclosure.

The level of fibulin 1 may also be considered when deciding the intensity of a renal disorder treatment. As a second configuration of the fourth aspect of the present disclosure, there is thus provided a method for determining the level of intensity of a treatment of a subject having a renal disorder, comprising the steps of:
a) measuring an amount of fibulin 1 in a sample from the subject to obtain a sample value;
b) comparing the sample value to a reference value; and if the sample value is higher than the reference value,
c1) concluding that said subject should be given a treatment of a first intensity; and
   if the sample value is lower than the reference value,
c2) concluding that said subject should be given a treatment of a second intensity,
   wherein the first intensity is higher than the first intensity.

The level of intensity may for example be measured as the average daily or weekly dose of a therapeutic agent given to the subject. A treatment of the first intensity may thus be applied more frequently or in higher individual doses than a treatment of the second intensity. The treatment of the first intensity may also comprise application of a more aggressive therapy than the treatment of the second intensity. For example, the treatment of the first intensity may be a combination treatment while the treatment of the second intensity is a mono-therapy. Yet another possibility is that the treatment of the first intensity is applied for a longer period than the treatment of the second intensity.

In an embodiment of the fourth aspect, c1) may thus be concluding that said subject should undergo treatment during a first period and c2) may be concluding that said subject should undergo treatment during a second period, wherein the first period is longer than the second period.

As a specific example, the renal disorder treatment may be renal replacement therapy, such as dialysis. Thus, dialysis or hemofiltration may be performed more regularly and/or for a longer period if high levels of fibulin 1 are detected.

In some embodiments, the above methods further comprise the measurement of the level(s) of one or more of the biomarkers presented under the fifth aspect below. In such embodiments, the mean or median sample values of fibulin 1 and/or the other biomarker is higher in the first group then the respective sample values in the second group. The other biomarker(s) may be detected in the same sample as fibulin 1 or in another sample from the same subject, preferably taken at the same time. The sample value(s) of the other biomarker(s) may be established and compared to a relevant sample value in the same manner as the sample value of fibulin 1. Thus, the various embodiments and examples herein related to fibulin 1 apply *mutatis mutandis* to any embodiment involving one of the other biomarkers. Also, as alternative configurations of the present disclosure, there are provided methods according to the above aspects wherein the level of one of the following biomarkers is measured: GC, SLC13A3, GDA, MACF1, MAPK3, RBP4, SFXN1 and BBOX1.

With the knowledge of the teachings of the present disclosure, the person skilled in the art may without undue burden select a relevant reference value for the methods of aspects one, three and four. For example, the skilled person may measure the amount of fibulin 1 protein in samples from a first group of subjects having a renal impairment or a certain renal disorder. Thereby the skilled person would obtain a first value, such as a mean or median value, corresponding to the presence of the renal impairment or the renal disorder. As a comparison, the skilled person may measure the amount of fibulin 1 in samples from a second group of healthy subjects, or at least subjects without renal impairments. Thereby the skilled person would obtain a second value, such as a mean or median value, corresponding to a (relatively) healthy condition. A value, which is lower or equal to the first value, but higher than the second value, can then be selected as the reference value.

The steps of the above methods may be performed by a single person, such as a lab technician, nurse or physician. However, a physician may also perform step c) and optionally step b) himself, while assigning the performance of step a) and optionally step b) to someone else, typically a lab technician or nurse. If the above methods involve more than one person, the persons may be situated at different locations and have different employers. For example, the physician may be employed by a hospital while the lab technician is employed by a lab. Samples and information may thus be transferred from one person and location to another within the framework of the methods of the present disclosure.

Regarding step a) of the methods of the present disclosure, an increase in the amount of fibulin 1 typically results in an increase in the sample value, and not the other way around. However, in some embodiments, the amount may correspond to any of a predetermined number of discrete sample values. In such embodiments, a first amount and a second, increased, amount may correspond to the same sample value. In any case, an increase in the amount fibulin 1 will not result in a decrease in the sample value in the context of the present disclosure.

However inconvenient, but in an equivalent fashion, the evaluated amounts may be inversely related to sample values if the qualification between steps b) and c) is inverted. For example, the qualification between steps b) and c) is inverted if the phrase "if the sample value is higher than the reference value" is replaced with "if the sample value is lower than the reference value".

The methods and uses of the present disclosure, except the methods of treatment, may unless otherwise stated be carried out entirely *ex vivo.* Thus, the sample of step a) of the above methods may be a previously obtained sample, which means that the actual step of obtaining the sample from the subject, i.e. the physical interaction with the subject, is not part of the methods.

The inventors have identified fibulin 1 in blood-derived samples as well as in urine (see the Examples below). Further, a correlation between high levels of fibulin 1 and renal impairment have been observed in such samples. Accordingly, the inventors believe that there may be a general correlation between levels of fibulin 1 and renal function in human samples. Thus, the sample of the above methods may for example be a tissue sample, such as a tissue extract. However, in preferred embodiments of the above methods, the sample is an optionally modified blood sample, such as an optionally diluted serum or plasma sample, or an optionally modified urine sample. The sample may for example have been modified by heat treated before the measurement of level of fibulin 1. An example of such a heat treatment is described in WO 2010/151180. In some embodiments, step a) of the methods of the above aspects may comprise obtaining blood from the subject and preparing serum or plasma from the obtained blood to facilitate the evaluation of step a). Step a) may also comprise diluting serum or plasma, e.g. in one or more buffers, at least 10 times, such as at least 50 times.

The subject of the above methods is preferably a mammalian subject, such as a human.

The skilled person should recognize that the usefulness of the above methods or other aspects of the present disclosure is not limited to the quantification of any particular variant of fibulin 1, as long as it is encoded by the relevant gene and presents the relevant pattern of expression.

As a non-limiting example, the fibulin 1 protein of the present disclosure may comprise a sequence selected from:
i) SEQ ID NO:1;
ii) SEQ ID NO:2;
iii) SEQ ID NO:3; and
iv) a sequence which is at least 85 % identical to SEQ ID NO:1, 2 or 3.

In some embodiments, sequence iv) above is at least 90 % identical, at least 91 % identical, at least 92 % identical, at least 93 % identical, at least 94 % identical, at least 95 % identical, at least 96 % identical, at least 97 % identical, at least 98 % identical or at least 99 % identical to SEQ ID NO:1, 2 or 3.

The term "% identical", as used in the context of the present disclosure, is calculated as follows. The query sequence is aligned to the target sequence using the CLUSTAL W algorithm (Thompson, J.D., Higgins, D.G. and Gibson, T.J., Nucleic Acids Research, 22: 4673-4680 (1994)). The amino acid residues at each position are compared, and the percentage of positions in the query sequence that have identical correspondences in the target sequence is reported as % identical. Also, the target sequence determines the number of positions that are compared. Consequently, in the context of the present disclosure, a query sequence that is shorter than the target sequence can never be 100 % identical to the target sequence. For example, a query sequence of 85 amino acid residues may at the most be 85 % identical to a target sequence of 100 amino acid residues.

In embodiments of the above methods, step a) may comprise contacting the sample with an affinity ligand that is, under the conditions of the contact, capable of selective interaction with fibulin 1.

An affinity ligand may be capable of selective interaction with a target in a first set-up, but not in a second. For example, an antibody may be capable of selective interaction with the target in a sandwich assay (see below), in which an optionally diluted urine, blood, serum or plasma sample containing the target is contacted with the antibody immobilized on a solid phase, while the same antibody is not capable of selective interaction with the target in a western blot experiment.

In the context of the present disclosure, "selective" interaction of e.g., an affinity ligand with its target or antigen means that the interaction is such that a distinction between selective and non-selective interaction becomes meaningful. The interaction between two proteins is sometimes measured by the dissociation constant. The dissociation constant describes the strength of binding (or affinity) between two molecules. Typically the dissociation constant between an antibody and its antigen is from 10⁻⁷ to 10⁻¹¹ M. However, high selectivity does not necessarily require high affinity. Molecules with low affinity (in the molar range) for its counterpart have been shown to be as selective as molecules with much higher affinity. In the case of the present disclosure, a selective interaction refers to the extent to which a particular method can be used to determine the presence and/or amount of a specific protein, the target protein, under given conditions in the presence of other proteins in a fluid sample of a naturally occurring or processed biological fluid (e.g. serum or plasma). In other words, selectivity is the capacity to distinguish between related proteins. "Specific" and "selective" are sometimes used interchangeably in the present description. For example, the specificity or selectivity of an antibody may be determined as in Examples, Section 2, below. Specificity and selectivity determinations are also described in Nilsson P et al. (2005) Proteomics 5:4327-4337.

Step a) may further comprise quantification of the interaction between the affinity ligand and fibulin 1 to obtain the sample value. Examples of such a quantification are discussed below.

As indicated above, step a) may for example comprise a sandwich assay.

In the context of the present disclosure, a "sandwich assay" refers to an assay comprising:
contacting the sample with a first affinity ligand (capture affinity ligand) immobilized to a solid support, which first affinity ligand is capable of selective interaction with fibulin 1 under the conditions of the contact;
removing unbound affinity ligand (e.g. washing the solid support); and
contacting the solid support with a second affinity ligand (detection affinity ligand) capable of interaction with fibulin 1 bound to the first affinity ligand, which second affinity ligand is directly or indirectly detectable.

Unbound affinity ligand may be removed again after the contact with the second affinity ligand (e.g. another washing) and the amount of bound second affinity ligand may then be detected and/or quantified.

The second affinity ligand may thus comprise a detectable label, such as a quantifiable label. Alternatively, the second affinity ligand may be detectable by an agent comprising the detectable label, such as the quantifiable label. For example, the second affinity ligand may be a goat antibody, and the agent may be a labeled anti-goat antibody. Several labels enabling detection and/or quantification are discussed below.

Since the antigen is recognized twice with intermediate removal of unspecific binding, the sandwich assay facilitates sensitive and quantifiable detection fibulin 1.

The sandwich assay of the present disclosure may for example be an ELISA sandwich assay, which is an assay for quantifying the amount of antigen in a sample, such as a blood-derived or urine-derived sample.

The skilled person may, without undue burden, implement a sandwich or ELISA assay in the above methods using the teachings of the present disclosure (ELISA is also further discussed below).

Further sandwich assay embodiments of the above methods are evident from the discussion about sandwich assays in connection with the kit aspect below.

It is regarded as within the capabilities of those of ordinary skill in the art to select or manufacture the proper affinity ligand and to select the proper format and conditions for detection and/or quantification. Nevertheless, examples of affinity ligands that may prove useful, as well as examples of formats and conditions for detection and/or quantification, are given below for the sake of illustration.

Thus, in embodiments of the present disclosure, the affinity ligand may be selected from the group consisting of antibodies, fragments thereof and derivatives thereof, i.e., affinity ligands based on an immunoglobulin scaffold. The antibodies and the fragments or derivatives thereof may be isolated. Antibodies comprise monoclonal and polyclonal antibodies of any origin, including murine, rabbit, human and other antibodies, as well as chimeric antibodies comprising sequences from different species, such as partly humanized antibodies, e.g., partly humanized mouse antibodies. Polyclonal antibodies are produced by immunization of animals with the antigen of choice. The polyclonal antibodies may be antigen-purified. Monoclonal antibodies of defined specificity can be produced using the hybridoma technology developed by Köhler and Milstein (Köhler G and Milstein C (1976) Eur. J. Immunol. 6:511-519). The antibody fragments and derivatives of the present disclosure are capable of selective interaction with the same antigen (e.g. fibulin 1 protein or fragments thereof) as the antibody they are fragments or derivatives of. Antibody fragments and derivatives comprise Fab fragments, consisting of the first constant domain of the heavy chain (CH1), the constant domain of the light chain (CL), the variable domain of the heavy chain (VH) and the variable domain of the light chain (VL) of an intact immunoglobulin protein; Fv fragments, consisting of the two variable antibody domains VH and VL (Skerra A and Plückthun A (1988) Science 240:1038-1041); single chain Fv fragments (scFv), consisting of the two VH and VL domains linked together by a flexible peptide linker (Bird RE and Walker BW (1991) Trends Biotechnol. 9:132-137); Bence Jones dimers (Stevens FJ et al. (1991) Biochemistry 30:6803-6805); camelid heavy-chain dimers (Hamers-Casterman C et al. (1993) Nature 363:446-448) and single variable domains (Cai X and Garen A (1996) Proc. Natl. Acad. Sci. U.S.A. 93:6280-6285; Masat L et al. (1994) Proc. Natl. Acad. Sci. U.S.A. 91:893-896), and single domain scaffolds like e.g., the New Antigen Receptor (NAR) from the nurse shark (Dooley H et al. (2003) Mol. Immunol. 40:25-33) and minibodies based on a variable heavy domain (Skerra A and Plückthun A (1988) Science 240:1038-1041).

In the context of the present disclosure, a "antigen-purified antibody" is a population of polyclonal antibodies which has been affinity purified on its own antigen, thereby separating such antigen-purified antibodies from other antiserum proteins and non-specific antibodies. This affinity purification results in antibodies that bind selectively to its antigen. In the Examples below, the polyclonal antisera are purified by a three-step immunoaffinity based protocol to obtain antigen-purified antibodies selective for the target protein (see Examples, Section 2).

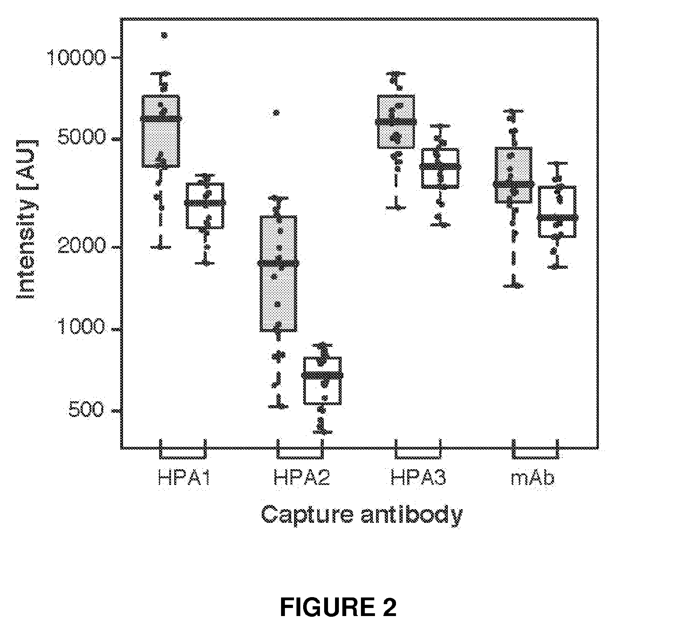

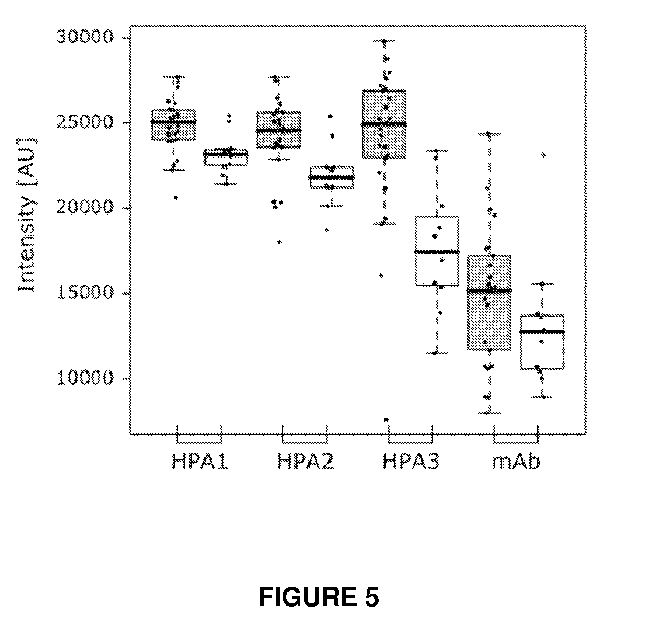

The fibulin 1 fragments SEQ ID NO:1 and 2 were designed to lack transmembrane regions to ensure efficient expression in *E*. *coli,* and to lack any signal peptide, since those are cleaved off in the mature protein. SEQ ID NO:1 and 2 were thus designed for immunizations. In addition, the protein fragments were designed to consist of a unique sequence with low sequence identity to other human proteins, to minimize cross reactivity of generated affinity reagents, and to be of a suitable size to allow the formation of conformational epitopes and still allow efficient cloning and expression in bacterial systems. Further, the antibodies capable of selective interaction with SEQ ID NO:1 or 2 appear to separate diseased subjects from "healthy" subjects more successfully than a commercial antibody binding another fibulin 1 epitope region (see figures 2 and 5). Accordingly, in the cases wherein the affinity ligand is an antibody or fragment or derivative thereof, the affinity ligand may be obtainable by a process comprising a step of immunizing an animal with a peptide whose amino acid sequence consists of sequence SEQ ID NO:1 or 2. For example, the immunization process may comprise primary immunization with the protein in Freund's complete adjuvant. Also, the immunization process may further comprise boosting at least two times, in intervals of 2-6 weeks, with the protein in Freund's incomplete adjuvant. Processes for the production of antibodies or fragments or derivatives thereof against a given target are known in the art.

Polyclonal and monoclonal antibodies, as well as their fragments and derivatives, represent the traditional choice of affinity ligands in applications requiring selective biomolecular recognition, such as in the detection and/or quantification of fibulin 1 protein according to the method aspects above. However, those of skill in the art know that, due to the increasing demand of high throughput generation of selective binding ligands and low cost production systems, new biomolecular diversity technologies have been developed during the last decade. This has enabled a generation of novel types of affinity ligands of both immunoglobulin as well as non-immunoglobulin origin that have proven equally useful as binding ligands in biomolecular recognition applications and can be used instead of, or together with, immunoglobulins.

The biomolecular diversity needed for selection of affinity ligands may be generated by combinatorial engineering of one of a plurality of possible scaffold molecules, and specific/selective affinity ligands are then selected using a suitable selection platform. The scaffold molecule may be of immunoglobulin protein origin (Bradbury AR and Marks JD (2004) J. lmmunol. Meths. 290:29-49), of non-immunoglobulin protein origin (Nygren PÅ and Skerra A (2004) J. Immunol. Meths. 290:3-28), or of an oligonucleotide origin (Gold L et al. (1995) Annu. Rev. Biochem. 64:763-797).

A large number of non-immunoglobulin protein scaffolds have been used as supporting structures in development of novel binding proteins. Non-limiting examples of such structures, useful for generating affinity ligands against fibulin 1 protein for use according to the present disclosure, are staphylococcal protein A and domains thereof and derivatives of these domains, such as protein Z (Nord K et al. (1997) Nat. Biotechnol. 15:772-777); lipocalins (Beste G et al. (1999) Proc. Natl. Acad. Sci. U.S.A. 96:1898-1903); ankyrin repeat domains (Binz HK et al. (2003) J. Mol. Biol. 332:489-503); cellulose binding domains (CBD) (Smith GP et al. (1998) J. Mol. Biol. 277:317-332; Lehtiö J et al. (2000) Proteins 41:316-322); γ crystallines (Fiedler U and Rudolph R, WO01/04144); green fluorescent protein (GFP) (Peelle B et al. (2001) Chem. Biol. 8:521-534); human cytotoxic T lymphocyte-associated antigen 4 (CTLA-4) (Hufton SE et al. (2000) FEBS Lett. 475:225-231; Irving RA et al. (2001) J. Immunol. Meth. 248:31-45); protease inhibitors, such as Knottin proteins (Wentzel A et al. (2001) J. Bacteriol. 183:7273-7284; Baggio R et al. (2002) J. Mol. Recognit. 15:126-134) and Kunitz domains (Roberts BL et al. (1992) Gene 121:9-15; Dennis MS and Lazarus RA (1994) J. Biol. Chem. 269:22137-22144); PDZ domains (Schneider S et al. (1999) Nat. Biotechnol. 17:170-175); peptide aptamers, such as thioredoxin (Lu Z et al. (1995) Biotechnology 13:366-372; Klevenz B et al. (2002) Cell. Mol. Life Sci. 59:1993-1998); staphylococcal nuclease (Norman TC et al. (1999) Science 285:591-595); tendamistats (McConell SJ and Hoess RH (1995) J. Mol. Biol. 250:460-479; Li R et al. (2003) Protein Eng. 16:65-72); trinectins based on the fibronectin type III domain (Koide A et al. (1998) J. Mol. Biol. 284:1141-1151; Xu L et al. (2002) Chem. Biol. 9:933-942); and zinc fingers (Bianchi E et al. (1995) J. Mol. Biol. 247:154-160; Klug A (1999) J. Mol. Biol. 293:215-218; Segal DJ et al. (2003) Biochemistry 42:2137-2148).

The above-mentioned examples of non-immunoglobulin protein scaffolds include scaffold proteins presenting a single randomized loop used for the generation of novel binding specificities, protein scaffolds with a rigid secondary structure where side chains protruding from the protein surface are randomized for the generation of novel binding specificities, and scaffolds exhibiting a non-contiguous hyper-variable loop region used for the generation of novel binding specificities.

In addition to non-immunoglobulin proteins, oligonucleotides may also be used as affinity ligands. Single stranded nucleic acids, called aptamers or decoys, fold into well-defined three-dimensional structures and bind to their target with high affinity and specificity. (Ellington AD and Szostak JW (1990) Nature 346:818-822; Brody EN and Gold L (2000) J. Biotechnol. 74:5-13; Mayer G and Jenne A (2004) BioDrugs 18:351-359). The oligonucleotide ligands can be either RNA or DNA and can bind to a wide range of target molecule classes.

For selection of the desired affinity ligand from a pool of variants of any of the scaffold structures mentioned above, a number of selection platforms are available for the isolation of a specific novel ligand against a target protein of choice. Selection platforms include, but are not limited to, phage display (Smith GP (1985) Science 228:1315-1317), ribosome display (Hanes J and Plückthun A (1997) Proc. Natl. Acad. Sci. U.S.A. 94:4937-4942), yeast two-hybrid system (Fields S and Song O (1989) Nature 340:245-246), yeast display (Gai SA and Wittrup KD (2007) Curr Opin Struct Biol 17:467-473), mRNA display (Roberts RW and Szostak JW (1997) Proc. Natl. Acad. Sci. U.S.A. 94:12297-12302), bacterial display (Daugherty PS (2007) Curr Opin Struct Biol 17:474-480, Kronqvist N et al. (2008) Protein Eng Des Sel 1-9, Harvey BR et al. (2004) PNAS 101 (25):913-9198), microbead display (Nord O et al. (2003) J Biotechnol 106:1-13, WO01/05808), SELEX (System Evolution of Ligands by Exponential Enrichment) (Tuerk C and Gold L (1990) Science 249:505-510) and protein fragment complementation assays (PCA) (Remy I and Michnick SW (1999) Proc. Natl. Acad. Sci. U.S.A. 96:5394-5399).

Thus, in embodiments of the present disclosure, the affinity ligand may be a non-immunoglobulin affinity ligand derived from any of the protein scaffolds listed above, or an oligonucleotide molecule.

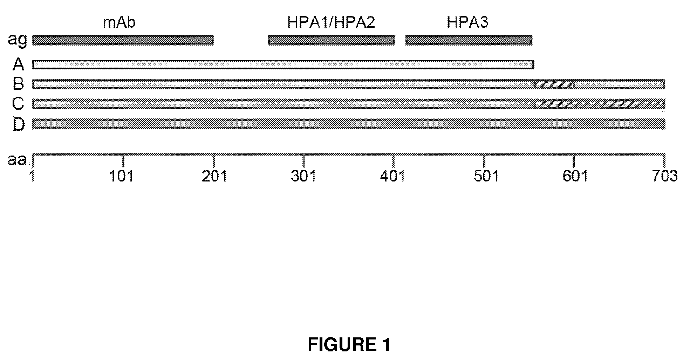

There are several splice variants of the fibulin 1 protein, and those believed to be the most common are SEQ ID NO:3-6 (see figure 1). The affinity ligand of the present disclosure may thus be capable of selective interaction with a peptide consisting of amino acid sequence SEQ ID NO:3, SEQ ID NO:4, SEQ ID NO:5 and/or SEQ ID NO:6. A single affinity ligand, such as the affinity ligand capable of selective interaction with SEQ ID NO:2 or SEQ ID NO:3, may thus be capable of recognizing all splice variants in question, which may be beneficial.

As mentioned above, SEQ ID NO:1 and SEQ ID NO:2 are associated with a number of benefits. Consequently, in embodiments of the present disclosure, the affinity ligand may be capable of selective interaction with a polypeptide consisting of amino acid sequence SEQ ID NO:1 or SEQ ID NO:2.

The detection and/or quantification of the affinity ligand capable of selective interaction with fibulin 1 may be accomplished in any way known to the skilled person for detection and/or quantification of binding reagents in assays based on biological interactions. Accordingly, any affinity ligand described above may be used to quantitatively and/or qualitatively detect the presence of fibulin 1. These "primary" affinity ligands may be labeled themselves with various markers or may in turn be detected by secondary, labeled affinity ligands to allow detection, visualization and/or quantification. This can be accomplished using any one or more of a multitude of labels, which can be conjugated to the affinity ligand capable of interaction with fibulin 1 or to any secondary affinity ligand, using any one or more of a multitude of techniques known to the skilled person, and not as such involving any undue experimentation.

Non-limiting examples of labels that can be conjugated to primary and/or secondary affinity ligands include fluorescent dyes or metals (e.g., fluorescein, rhodamine, phycoerythrin, fluorescamine), chromophoric dyes (e.g., rhodopsin), chemiluminescent compounds (e.g., luminal, imidazole) and bioluminescent proteins (e.g., luciferin, luciferase), haptens (e.g., biotin). A variety of other useful fluorescers and chromophores are described in Stryer L (1968) Science 162:526-533 and Brand L and Gohlke JR (1972) Annu. Rev. Biochem. 41:843-868. Affinity ligands can also be labeled with enzymes (e.g., horseradish peroxidase, alkaline phosphatase, beta-lactamase), radioisotopes (e.g., ³H, ¹⁴C, ³²P, ³⁵S or ¹²⁵I) and particles (e.g., gold). In the context of the present disclosure, "particles" refer to particles, such as metal particles, suitable for labeling of molecules. Further, the affinity ligands may also be labeled with fluorescent semiconductor nanocrystals (quantum dots). Quantum dots have superior quantum yield and are more photostable compared to organic fluorophores and are therefore more easily detected (Chan et al. (2002) Curr Opi Biotech. 13: 40-46). The different types of labels can be conjugated to an affinity ligand using various chemistries, e.g., the amine reaction or the thiol reaction. However, other reactive groups than amines and thiols can be used, e.g., aldehydes, carboxylic acids and glutamine.

The method aspects above may be put to use in any of several known formats and set-ups, of which a non-limiting selection is discussed below.

The method of visualization of labels on the affinity ligand may include, but is not restricted to, fluorometric, luminometric and/or enzymatic techniques. Fluorescence is detected and/or quantified by exposing fluorescent labels to light of a specific wavelength and thereafter detecting and/or quantifying the emitted light in a specific wavelength region. The presence of a luminescently tagged affinity ligand may be detected and/or quantified by luminescence developed during a chemical reaction. Detection of an enzymatic reaction is due to a color shift in the sample arising from a chemical reaction. Those of skill in the art are aware that a variety of different protocols can be modified in order for proper detection and/or quantification.

One available method for detection and/or quantification of fibulin 1 is by linking the affinity ligand to an enzyme that can then later be detected and/or quantified in an enzyme immunoassay (such as an EIA or ELISA). Such techniques are well established, and their realization does not present any undue difficulties to the skilled person. As discussed above, the biological sample is brought into contact with a solid material or with a solid material conjugated to an affinity ligand against fibulin 1 in such methods. The affinity ligand-antigen complex is then indirectly or directly detected with an enzymatically labeled secondary affinity ligand. Following this, an appropriate substrate is brought to react in appropriate buffers with the enzymatic label to produce a chemical moiety, which for example is detected and/or quantified using a spectrophotometer, fluorometer, luminometer or by visual means.

As stated above, primary and any secondary affinity ligands can be labeled with radioisotopes to enable detection and/or quantification. Non-limiting examples of appropriate radiolabels in the present disclosure are ³H, ¹⁴C, ³²P, ³⁵S or ¹²⁵I. The specific activity of the labeled affinity ligand is dependent upon the half-life of the radiolabel, isotopic purity, and how the label has been incorporated into the affinity ligand. Affinity ligands are preferably labeled using well-known techniques (Wensel TG and Meares CF (1983) in: Radioimmunoimaging and Radioimmunotherapy (Burchiel SW and Rhodes BA eds.) Elsevier, New York, pp 185-196). A thus radiolabeled affinity ligand can be used to visualize the fibulin 1 protein by detection of radioactivity. Radionuclear scanning with e.g., gamma camera, magnetic resonance spectroscopy or emission tomography function for detection *in vivo* and *in vitro,* while gamma/beta counters, scintillation counters and radiographies are also used *in vitro.*

When diagnosing the renal function of a subject, it may be beneficial to detect fibulin 1 and at least one more biomarker that may be used when diagnosing a renal impairment. Serum Creatinine (see e.g. Anal. Chem., 2000, 72 (5), pp 916-921), Kidney Injury Molecule-1 (KIM-1), Serum Cystatin C, Liver Fatty Acid Binding Protein (L-FABP), alpha-GST, pi-GST, Urinary Collagen IV, Alpha-1-Microglobulin, Beta-2-Microglobulin, Calbindin, Clusterin, Connective Tissue Growth Factor, Glutathione S-Transferase alpha, Urinary albumin, Microalbumin, Neutrophil Gelatinase-Associated Lipocalin (NGAL), Osteopontin, Tamm-Horsfall Urinary Glycoprotein, Tissue Inhibitor of Metalloproteinases 1, Trefoil Factor 3 (TFF3) and Vascular Endothelial Growth Factor (VEGF) are examples of such biomarkers. Thus, as a fifth aspect of the present disclosure there is provided a kit comprising:
a) a quantifiable affinity ligand capable of selective interaction with a fibulin 1 protein;
b) reagents necessary for quantifying the amount of the quantifiable affinity ligand of a);
a') a quantifiable affinity ligand capable of selective interaction with Serum Creatinine, KIM-1, Cystatin C, L-FABP, alpha-GST, pi-GST, Urinary Collagen IV, Alpha-1-Microglobulin, Beta-2-Microglobulin, Calbindin, Clusterin, Connective Tissue Growth Factor, Glutathione S-Transferase alpha, Urinary albumin, Microalbumin, NGAL, Osteopontin, Tamm-Horsfall Urinary Glycoprotein, Tissue Inhibitor of Metalloproteinases 1, TFF3 or VEGF; and
b') reagents necessary for quantifying the amount of the quantifiable affinity ligand of a'),
wherein the reagents of b) and b') may be the same or different.

In a preferred embodiment, the quantifiable affinity ligand of a') is capable of selective interaction with Serum Creatinine, KIM-1, Cystatin C, Urinary albumin, Beta-2-Microglobulin, Clusterin or TFF3.

In a particularly preferred embodiment, the quantifiable affinity ligand of a') is capable of selective interaction with Serum Creatinine, KIM-1 or Cystatin C.

As presented under Examples below, the inventors have identified a number of additional biomarkers that also may be correlated to a renal condition. Accordingly, it may be beneficial to detect fibulin 1 and one or more of these additional biomarkers when diagnosing the renal function of a subject. As a configuration of the fifth aspect, there is thus provided a kit comprising:
a) a quantifiable affinity ligand capable of selective interaction with a fibulin 1 protein;
b) reagents necessary for quantifying the amount of the quantifiable affinity ligand of a);
a') a quantifiable affinity ligand capable of selective interaction with GC, SLC13A3, GDA, MACF1, MAPK3, RBP4, SFXN1 or BBOX1; and
b') reagents necessary for quantifying the amount of the quantifiable affinity ligand of a'),
wherein the reagents of b) and b') may be the same or different.

In embodiments, the kits of the fifth aspect comprise at least two antibodies capable of selective interaction with at least two of the biomarkers of a').

Embodiments of the seventh aspect below apply *mutatis mutandis* to the fifth aspect.

Also, it is evident from the above, that an immobilized affinity ligand capable of selective interaction with fibulin 1 may find various diagnostic applications. Thus, as a sixth aspect of the present disclosure, there is provided a product comprising an affinity ligand capable of selective interaction with fibulin 1, which affinity ligand is immobilized onto a solid support. The solid support may for example be a plate for a sandwich assay or a bead for a bead-based assay. Further embodiments of the solid support are discussed above in connection with the method aspects and below in connection with kit aspect.

Various embodiments of the (quantifiable) affinity ligand of the fifth or sixth aspect are described above in connection with the method aspects.

Products for carrying out the above methods may advantageously be comprised in a kit.

As a seventh aspect of the present disclosure, there is thus provided a kit comprising:
a) a first affinity ligand capable of selective interaction with a fibulin 1 protein;
b) a quantifiable second affinity ligand capable of interaction with the fibulin 1 protein when bound to the first affinity ligand; and optionally
c) a solid support onto which the first affinity ligand is immobilized or which is prepared for immobilization of the first affinity ligand.

In embodiments of the seventh aspect, the first and second affinity ligand may independently be any one of the affinity ligands described above in connection with the method aspects.

Further, the quantifiable second affinity ligand is preferably capable of selective interaction with fibulin 1 when bound to the first affinity ligand. However, this is not necessary; only one of the affinity ligands of the kit has to be capable of selective interaction with fibulin 1.

Various components of the kit may be selected and specified as described above in connection with the method aspects of the present disclosure. The kit may also contain various auxiliary substances other than affinity ligands, to enable the kit to be used easily and efficiently. The kit may thus further comprise auxiliary substances selected from sample diluents, wash buffers, standard solutions containing fibulin 1 and substrate for measuring enzyme activity in cases where an enzyme is used as a label.

The standard solution(s) may be used for making a standard curve. The kit may thus comprise a standard solution of one single concentration (to be diluted to different concentrations by the user) or two or more standard solutions of different concentrations.

The kit may also contain a reference sample comprising a known amount of fibulin 1. The amount of fibulin 1 in the reference sample may correspond to a reference value employed in a method according to one of the above aspects. The reference sample may for example comprise plasma or serum.

In some embodiments, the first affinity ligand interacts with a fibulin 1 amino acid sequence that is different from the fibulin 1 amino acid sequence that the quantifiable second affinity ligand interacts with. An assay in which two separate epitopes or epitope regions must be recognized to generate a response/ signal increases the specificity by reducing the number of false positives.

For example, the first affinity ligand may be capable of selective interaction with a peptide whose amino acid sequence consist of one of SEQ ID NO:1 and SEQ ID NO:2. Further, the quantifiable second affinity ligand may be capable of selective interaction with a peptide whose amino acid sequence consist of the other one of SEQ ID NO:1 and SEQ ID NO:2.

To facilitate the quantification, the quantifiable second affinity ligand may be labeled with a quantifiable label. Alternatively, the kit may further comprise an agent capable of binding the quantifiable second affinity ligand, which agent is labeled with a quantifiable label. Using such an agent may be beneficial since it may be common to different quantifiable second affinity ligands recognizing different antigens.

In some embodiments, the quantifiable second affinity ligand may be an antibody and the agent an antibody capable of binding the Fc region of the quantifiable second affinity ligand. For example, the quantifiable second affinity ligand may be a goat antibody and the agent a labeled anti-goat antibody. Similarly, the quantifiable second affinity ligand may be a mouse antibody and the agent a labeled anti-mouse antibody.

The quantifiable label may for example be selected from the group consisting of fluorescent dyes and metals, chromophoric dyes, chemiluminescent compounds and bioluminescent proteins, enzymes, radioisotopes, particles and quantum dots. Such labels are further discussed above in connection with the method aspects.

The enzyme, which normally implies that the kit is an ELISA kit, may be selected from horseradish peroxidase, alkaline phaosphatase, beta-D-galactosidase and beta-lactamase.

Further, when the quantifiable label is an enzyme, the kit may further comprise a chromogenic ELISA substrate.

The solid support may for example comprise wells in which the first affinity ligand is immobilized or which are prepared for immobilization of the first affinity ligand. Such a solid support may be adapted for a sandwich assay, such as an ELISA sandwich assay. Consequently, the first affinity ligand may be immobilized on a (microwell) plate adapted for an ELISA plate reader.

Alternatively, the solid support may be beads, such as magnetic beads, agarose beads or sepharose beads, to which the first affinity ligand is coupled. Such beads facilitate a miniaturized protocol requiring only small amounts of sample and affinity ligand. Luminex instrumentation, which is commercially available and well-known to the skilled person, may be employed for such a miniaturized protocol. Examples of commercially available beads are Dynabeads and Microsperes from Luminex-Corp.

The kit may also comprise instructions for the quantification of fibulin 1 in biological samples, such as samples derived from blood or urine.

As an eighth aspect of the present disclosure, there is provided a use of fibulin 1 as a diagnostic marker for renal impairment. Various renal impairments are discussed above. In an embodiment, the use is as a diagnostic marker for renal impairment in a clinical trial of a treatment. Further, the fibulin 1 of the eighth aspect may for example be provided in a blood-derived or urine-derived sample.

In the context of the present disclosure, a "diagnostic marker" refers to a biological entity which presence or absence correlates with a medical condition. The marker may thus be a biomarker, such as a human protein.

As a ninth aspect of the present disclosure, there is provided a use of an affinity ligand capable of selective interaction with fibulin 1 as a diagnostic agent for a renal impairment. Various embodiments of such an affinity ligand are discussed above in connection with the method aspects. In an embodiment of the ninth aspect, the affinity ligand is immobilized onto a solid support. Various embodiments of such an immobilization is discussed above.

In the context of the present disclosure, "diagnostic agent" refers to an agent having at least one property being valuable in an establishment of a diagnosis. For example, the diagnostic agent may be capable of selective interaction with the diagnostic marker.

The uses of aspects eight and nine may for example be *ex vivo* uses.

It follows from the above that the uses of aspects eight and nine may be particularly well suited for clinical trials of treatments.

### Examples

### 1. Generation of antigen

### a) Materials and methods

Suitable fragments of the target protein encoded by the Ensembl Gene ID ENSG00000077942 (Ensemble release 62, April 2011) were selected using bioinformatic tools with the human genome sequence as template (Berglund, L. et al (2008) Proteomics 8: 2832-9, Ensembl, www.ensembl.org). The fragments were used as templates for production of a 137 amino acid long fragment, corresponding to amino acids 266-402 (SEQ ID NO:1) of the fibulin 1 protein (SEQ ID NO:3; Ensembl entry no. ENSP00000331544), and a 143 amino acid long fragment corresponding to amino acids 417-559 (SEQ ID NO:2) of the fibulin 1 protein (SEQ ID NO:3; Ensembl entry no. ENSP00000331544).

The fibulin 1 protein exists in several splice variants. Both SEQ ID NO:1 and SEQ ID NO:2 are within the region where the most common splice variants (SEQ ID NO:3-6) overlap (Figure 1).

A first fragment of the fibulin 1 gene transcript containing nucleotides 796-1206, of EnsEMBL entry number ENST00000327858 (SEQ ID NO:7), was isolated by a Superscript® III One-Step RT-PCR amplification kit with Platinum® Taq (Invitrogen) and a human total RNA pool panel as template (Human Total RNA, BD Biosciences Clontech) (forward primer: TGTGAGAGTGGTATTCATAAC (SEQ ID NO:8), reverse primer:
CTCGTTGACATCGACACAC (SEQ ID NO:9).

A second fragment of the fibulin 1 gene transcript containing nucleotides 1248-1677, of EnsEMBL entry number ENST00000327858 (SEQ ID NO:7), was isolated by a Superscript® III One-Step RT-PCR amplification kit with Platinum® Taq (Invitrogen) and a human total RNA pool panel as template (Human Total RNA, BD Biosciences Clontech) (forward primer: AACACGCTGGGCTCCTAC (SEQ ID NO:10), reverse primer:
GTTCTCAGGGCACTCGAAG (SEQ ID NO:11)

Also, flanking restriction sites Notl (5') and Ascl (3') were introduced into the fragments through the PCR amplification primers, to allow in-frame cloning into the expression vector. In a second PCR reaction a biotinylated primer was coupled to the 3' end, and a non-biotinylated primer to the 5' end, of the isolated fragment using the *Pfx50*™ DNA Polymerase kit (Invitrogen). The resulting biotinylated PCR product was immobilized onto Dynabeads M280 Streptavidin (NorDiag) (Larsson M et al (2000) J. Biotechnol. 80:143-157). The fragments were released from the solid support by Notl-Ascl digestion (New England Biolabs), ligated into the pAff8c vector (Larsson M *et al*, *supra*) in frame with a dual affinity tag consisting of a hexahistidyl tag for immobilized metal ion chromatography (IMAC) purification and an immunopotentiating and solubilizing albumin binding protein (ABP) from streptococcal protein G (Sjölander A et al (1997) J. Immunol. Methods 201:115-123; Ståhl S et al (1999) Encyclopedia of Bioprocess Technology: Fermentation, Biocatalysis and Bioseparation (Fleckinger MC and Drew SW, eds) John Wiley and Sons Inc., New York, pp 49-63), and transformed into *E. coli* Rosetta(DE3) cells (Novagen). The sequences of the clones were verified by dye-terminator cycle sequencing of plasmid DNA using BigDye® Terminator v3.1 Cycle Sequencing Kit (Life Technologies).

Rosetta(DE3) cells (Novagen, Merck) harboring the expression vector were inoculated in 100 ml 30 g/I tryptic soy broth (Merck KGaA) supplemented with 5 g/I yeast extract (Merck KGaA), 20 mg/l chloramphenicol (Sigma-Aldrich), and 50 mg/l kanamycin (Sigma-Aldrich) by addition of 1 ml of an overnight culture in the same culture medium. The cell culture was incubated in a 1 liter shake flask at 37 °C and 150 rpm until the optical density at 600 nm reached 0.5-1.5. Protein expression was then induced by addition of isopropyl-β-D-thiogalactopyranoside (IPTG) (Apollo Scientific) to a final concentration of 1 mM, and the incubation was continued overnight at 25 °C and 150 rpm. The cells were harvested by centrifugation at 2400 g for 8 minutes, and the pellet was re-suspended in 5 ml lysis buffer (7 M guanidine hydrochloride, 47 mM Na₂HPO₄, 2.65 mM NaH₂P0₄, 10 mM Tris-HCl, 100 mM NaCl, 20 mM β-mercaptoethanol; pH = 8.0) and incubated for 2 hours at 37 °C and 150 rpm. After centrifugation at 35300 g, the supernatant containing the denatured and solubilized protein was collected.

The His₆-tagged fusion protein was purified by immobilized metal ion affinity chromatography (IMAC) on a column with 1 ml Talon® metal (Co²⁺) affinity resin (Invitro) using an automated protein purification procedure (Steen J et al (2006) Protein Expr. Purif. 46:173-178) on an ASPEC XL4™ (Gilson). The resin was equilibrated with 20 ml denaturing washing buffer (6 M guanidine hydrochloride, 46.6 mM Na₂HPO₄, 3.4 mM NaH₂PO₄, 300 mM NaCl, pH 8.0-8.2). Clarified cell lysate was then added to the column. Thereafter, the resin was washed with a minimum of 31.5 ml washing buffer prior to elution in 2.5 ml elution buffer (6 M urea, 50 mM NaH₂PO₄, 100 mM NaCl, 30 mM acetic acid, 70 mM Na-acetate, pH 5.0). The eluted material was fractioned in three pools of 500, 700 and 1300 µl. The 700 µl fraction, the fraction with the highest antigen concentration, and the pooled 500 and 1300 µl fractions were stored for further use.

The antigen fraction was diluted to a final concentration of 1 M urea with phosphate buffered saline (PBS; 1.9 mM NaH₂PO₄, 8.1 mM Na₂HPO₄, 154 mM NaCl) followed by a concentration step to increase the protein concentration using Vivapore 10/20 ml concentrator with molecular weight cut off at 7500 Da (Sartorius stedim). The protein concentration was determined using a bicinchoninic acid (BCA) micro assay protocol (Pierce) with a bovine serum albumin standard according to the manufacturer's recommendations. The protein purity was analyzed by SDS-PAGE using Criterion 15 % Tris-HCl gel system (BioRad) and the molecular weight of the protein product was verified by mass spectrometry (LC-ESI-MS).

### b) Results

Gene fragments corresponding to amino acid SEQ ID NO:1 and SEQ ID NO:2 were successfully isolated by RT-PCR from a human RNA pool using target-specific primers. The fragments code for amino acids 266 to 402 and amino acids 417-559 of the target protein fibulin 1 (SEQ ID NO:3-6). The 137 and 143 amino acid long fragments (SEQ ID NO:1 and SEQ ID NO:2) of the target protein (SEQ ID NO:3-6) were selected in a region having no predicted transmembrane region, to ensure efficient expression in *E. coli.* Any predicted signal peptide was also avoided in the selection step, since those are cleaved off in the mature protein. In addition, the protein fragments were selected to consist of a unique sequence with low sequence identity to other human proteins, to minimize cross reactivity of generated affinity reagents.

Clones encoding the correct amino acid sequences were identified, and, upon expression in *E. coli*, protein fragments of the correct size were produced and subsequently purified using immobilized metal ion chromatography. After dilution of the eluted samples to a final concentration of 1 M urea and concentration of the samples to 1 ml, the concentration of the protein fragments were determined to be 16.2 mg/ml (SEQ ID NO:1) and 15.2 mg/ml (SEQ ID NO:2) and evaluated as pure according to the purity analysis.

### 2. Generation of antibodies

### a) Materials and methods

The purified fibulin 1 fragments (SEQ ID NO:1 and SEQ ID NO:2) as obtained above were used as antigen to immunize separate rabbits. Rabbits were immunized intramuscularly with 200 µg of antigen in Freund's complete adjuvant as the primary immunization, and boosted three times in four week intervals with 100 µg antigen in Freund's incomplete adjuvant.

Antisera from the immunized animals were purified by a three-step immunoaffinity based protocol (Agaton C et al (2004) J. Chromatogr. A 1043:33-40; Nilsson P et al (2005) Proteomics 5:4327-4337). In the first step, 12 ml of total antiserum was buffered with 10x PBS to a final concentration of 1x PBS (1.9 mM NaH₂PO₄, 8.1 mM Na₂HPO₄, 15 mM NaCl), filtered using a 0.45 µm pore-size filter (Acrodisc®, Life Science) and applied to an affinity column containing 5 ml N-hydroxysuccinimide-activated Sepharose™ 4 Fast Flow (GE Healthcare) coupled to the dual affinity tag protein His₆-ABP (a hexahistidyl tag and an albumin binding protein tag) expressed from the pAff8c vector and purified in the same way as described above for the antigen protein fragments. In the second step, the flow-through, depleted of antibodies against the dual affinity tag His₆-ABP, was loaded at a flow rate of 0.7 ml/min on 1 ml Hi-Trap NHS-activated HP columns (GE Healthcare) coupled with the respective fibulin 1 protein fragments used as antigen for immunization (SEQ ID NO:1 and SEQ ID NO:2). The His₆-ABP protein and the protein fragment antigens were coupled to the NHS activated matrix as recommended by the manufacturer. Unbound material was washed away with 1x PBST (1x PBS, 0.1 % Tween20, pH 7.25), and captured antibodies were eluted using a low pH glycine buffer (0.2 M glycine, 1 mM EGTA, pH 2.5). The eluted antibody fractions were collected automatically, and loaded onto two 5 ml HiTrap™ desalting columns (GE Healthcare) connected in series for efficient buffer exchange in the third step. The second and third purification steps were run on the ÄKTAxpress™ platform (GE Healthcare). The antigen purified polyclonal antibodies were eluted with PBS buffer, supplemented with glycerol and NaN₃ to final concentrations of 50 % and 0.02 %, respectively, for long term storage at -20 °C (Nilsson P et al (2005) Proteomics 5:4327-4337).

The specificity and selectivity of the affinity purified antibody fractions were analyzed by binding analysis against the antigen itself and against 383 other human protein fragments in a protein array set-up (Nilsson P et al (2005) Proteomics 5:4327-4337). The protein fragments were diluted to 40 µg/ml in 0.1 M urea and 0,05 M sodium carbonate-bicarbonate buffer with 100 µg/mL BSA (BSA Cohn fraction V, protease free, Saveen Werner AB) and 50 µl of each were transferred to the wells of a 384-well spotting plate. The protein fragments were spotted in single spots and immobilized onto epoxy slides (Epoxide Coated Slide, Corning) using a non-contact piezoelectric arrayer (GeSim Nano-plotter 2). The slide was baked in 37°C for 12 hours and then blocked with BSA in 1xPBST, 30 mg/mL, for one hour. A 16-well incubation chamber (Schleicher & Schuell) was applied to the glass and inserted into a chip holder (Chip Clip^{™}, Schleicher & Schuell) before the affinity purified antibodies were added (diluted in 1x PBST to an appropriate concentration) and incubated on a shaker for 60 min. Affinity tag-specific IgY antibodies were co-incubated with the affinity purified antibodies in order to quantify the amount of protein in each spot. The slide was washed with 1x PBST three times for 15 min and 1x PBS once for 15 min. Secondary antibodies (goat anti-rabbit antibody conjugated with Alexa 647 and goat antichicken antibody conjugated with Alexa 555, Molecular Probes) were diluted 1:60000 to 30 ng/ml in 1x PBST and incubated for 60 min. After the same washing procedure, as for the first incubation, the slide was spun dry and scanned (G2565BA array scanner, Agilent), thereafter images were quantified using image analysis software (GenePix 5.1, Axon Instruments).

### b) Results

The quality of polyclonal antibody preparations has proven to be dependent on the degree of stringency in the antibody purifications, and it has previously been shown that depletion of antibodies directed against epitopes not originated from the target protein is necessary to avoid cross-reactivity to other proteins and background binding (Agaton C et al (2004) J. Chromatogr. A 1043:33-40). Thus, a protein microarray analysis was performed to ensure that antigen purified polyclonal antibodies of high specificity had been generated by depletion of antibodies directed against the His₆-tag as well as of antibodies against the ABP-tag.

To quantify the amount of protein in each spot of the protein array, a two-color dye labeling system was used, with a combination of primary and secondary antibodies. Tag-specific IgY antibodies generated in hen were detected with a secondary goat anti-hen antibody labeled with Alexa 555 fluorescent dye. The specific binding of the rabbit affinity purified antibodies to its antigen on the array was detected with a fluorescently Alexa 647 labeled goat anti-rabbit antibody. Each protein fragment was spotted in duplicates. The protein array analysis shows that the antigen purified antibodies against fibulin 1 are highly selective to the correct protein fragment and have a very low affinity for all other protein fragments analyzed on the array resulting in a low background.

### 3. Protein profiling

Three affinity purified polyclonal antibodies, obtained as described in Example 2 and denoted HPA1, HPA2 (both binding within SEQ ID NO:1), and HPA3 (binding within SEQ ID NO:2), as well as a mouse monoclonal antibody against another region of the fibulin 1 protein (sc-25281, Santa Cruz Biotechnology) (see Figure 1), were coupled to carboxylated beads (COOH Microspheres, Luminex-Corp.) according to the following procedure: A number of 5x105 magnetic beads per bead-ID (MagPlex microspheres, Luminex Corp.) where distributed into flat bottomed 96-well plates (Greiner BioOne) and washed with 0.1 M NaH2PO4 buffer (pH 6.2). Beads were activated by 0.5 mg N-hydroxysuccinimide (Pierce) and 0.5 mg 1-ethyl-3-(3-dimethylaminopropyl)-carbodiimide (Pierce) in 100 µl phosphate buffer. Upon a 20 min activation and subsequent washing in 0.05 M MES pH 5.0, 1.6 µg of antibody in 100 µl MES-buffer was added and incubated for 2 hours in room temperature. Additional bead identities were functionalized, either with 5 µg of recombinant albumin binding protein 18 (produced in-house), with 1.6 µg of rabbit IgG (Bethyl), or without addition of protein. After 2 h incubation, the beads where washed twice in PBST (PBS, 0,05% Tween20) and stored in plates in 100 µl of a gelatin based buffer (Blocking Reagent for ELISA, Roche) with the addition of NaN3. Suspension bead arrays (SBAs) were then prepared by combining equal volumes of each bead identity. After volume adjustment and sonicating for 5 min (Branson Ultrasonic Corp.) the bead arrays were stored at 4°C. The coupling efficiency of the antibodies was evaluated by an anti-rabbit IgG conjugated to R-phycoerythrin (Jackson Immunoresearch).

Plasma samples where aliquoted into 96-well plates in a randomized order including triplicates of 6 samples. To evaluate effects of repeated thawing and freezing of the samples, 3 samples that had been subjected to 3, 6 and 9 repeated freeze-thaw cycles were also included in the sample layout. For this purpose, samples had been thawed at room temperature from -80°C for about 30 min before beeing frozen again at -80°C. The samples were labeled with biotin, which involved the following: Samples were heated at 56°C for 30 minutes (PCR-block, BioRad) before diluted 1:10 in phosphate buffered saline (PBS) and labeled with 20 µg NHS-PEG4-biotin (Pierce) per 3 µl crude plasma for 2 h at 4°C. The labeling reaction was stopped by the addition of 12.5 µl of 0.5 M Tris-HCl (pH 8.0, Sigma) and the samples were stored in -20°C until further usage.

Upon analysis, the 1:10-diluted biotinylated plasma samples were thawed at room temperature, diluted 1:50 in assay buffer (0.5% w/v polyvinylalcohol (Sigma), 0.8% w/v polyvinylpyrrilidone (Sigma) and 0.1% w/v casein (Sigma) supplemented with 10% v/v rabbit IgG (Bethyl)) yielding a total sample dilution of 1:500. A heat treatment of 56°C for 30 min was performed and 45 µl sample where transferred to a 96-well assay plate (Greiner BioOne), mixed with 5 µl bead mixture and incubated over night at room temperature on gentle shaking (Thermomixer, Eppendorf). The beads were then washed in 3x 100 µl PBST using a liquid handler system (PlateMate 2x2, Matrix) and incubated in 50 µl 0.4 M paraformaldehyde in PBS. After 10 min, the beads were washed as noted above prior the addition of 50 µl streptavidin conjugated R-phycoerythrin (0.5 µg/ml, Invitrogen) and incubated for 20 min. After washing as above, 100 µl PBST was added for the read out performed by a Luminex Lx200 instrument, set to count at least 50 beads per ID and well. Every data point acquired was reported as the median fluorescence intensity (MFI) in arbitrary units (AU) of all beads of each identity within one reaction well.

### 4. Glomerulonephritis, cohort I

### a) Materials and methods

Plasma samples were obtained from Binding Site Inc. Lithium heparin plasma samples were collected from 20 consenting individuals with renal glomerulonephritis (GN). All patients were diagnosed based on ultrasound and/or X-ray imaging, supported with a CT scan for a smaller portion of the patients. In addition, plasma samples were collected from 20 healthy individuals matched in age, gender and body mass index. All samples were stored at -80°C until use, and then analyzed as described in Example 3.

### b) Results

Fibulin-1 was targeted by the three antibodies obtained as described in Example 2 (denoted HPA1-3), and the mouse monoclonal anti-fibulin 1 antibody (sc-25281, Santa Cruz Biotechnology). All antibodies could separate the glomerulonephritis (GN) patients from matched controls by measuring fibulin 1 (Figure 2). There was a higher level of fibulin 1 protein in plasma from GN patients (grey boxes) than in plasma from healthy individuals (white boxes). Mean values and 95% confidence intervals of the ratios between the sample group medians are presented with corresponding p-values under Experiment 1 in Table 1. The mean of the ratio obtained here was 1.4 with a 95% confidence interval of 1.1 to 1.6 with an associated p-value of 0.007.

A hierarchical cluster analysis of the protein profiles from processed samples and the rest of the technical replicates showed that regardless of freeze thaw cycle, the samples show protein profiles that cluster according to individual. The overall variation in the freeze-thawed samples was found to be in the same range as for the technical replicates.

**Table 1. Discriminatory capacity of fibulin 1 in glomerulonephritis case control comparisons.**

| **Antibody** | **Experiment 1** | | **Experiment 2** | |
|---|---|---|---|---|
| | P-value | Mean of ratio GN/ctrl (95% conf. interval) | P-value | Mean of ratio GN/ctrl (95% conf. interval) |
| HPA1 | 0.0003 | 1.6 (1.3 - 1.9) | 0.0003 | 1.6 (1.3 - 2.0) |
| HPA2 | 0.00001 | 1.7 (1.3 - 2.2) | 0.000005 | 1.8 (1.5 - 2.3) |
| HPA3 | 0.004 | 1.3 (1.1 - 1.5) | 0.002 | 1.2 (1.1 - 1.4) |

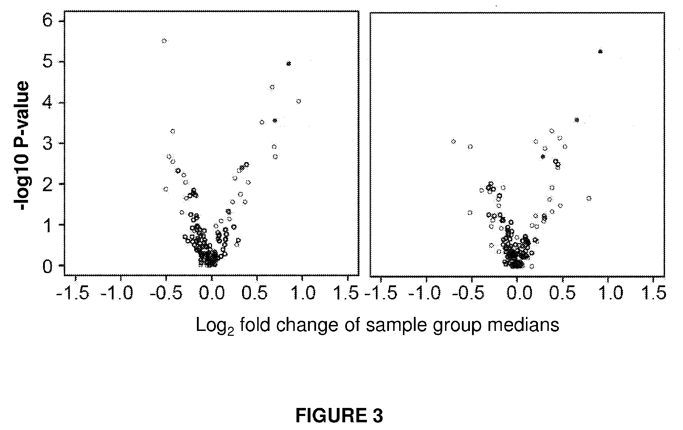

Eight other proteins were also identified, among 129 screened in the present cohort, as potential markers for renal impairment using appropriate antibodies obtained as described in Example 2. GC, SLC13A3, GDA, MACF1, MAPK3, RBP4, SFXN1 and BBOX1 have been shown by the inventors to discriminate between diseased patients and healthy controls in the experiment presented in Figure 3 or in another similar experienced based on another cohort.

### 5. Technical validation of antibodies by Western Blot Analysis

### a) Materials and methods

Plasma samples were diluted 1:60 into PBS-glycerol and incubated with SDS reducing buffer in 70°C for 10 minutes. Gel separation was performed in a 4-12% BisTris gel (Invitrogen) in MOPS-based buffer (2.5 mM MOPS, 2.5 mM Tris Base, 0.005% SDS, 0.05 mM EDTA at 200 V for 1 hour and 15 minutes in room temperature followed by electroblotting onto PVDF membranes (0.45 µm pore size, Invitrogen) in a transfer buffer (25 mM bicine, 25 mM BisTris, 1 mM EDTA, 10% v/v EtOH) at 30 V for 3 hours in 4°C. Transfers were confirmed with Ponceau stain (Pierce) and membranes were stored dry. Upon usage, membranes were soaked in 95% ethanol and blocked in 5% w/v milk powder and 1% v/v Tween20 in Tris-buffered saline (TBS) for 1 h at room temperature. The blocking buffer was exchanged and membranes were incubated with anti-fibulin-1 antibodies, obtained as described in Example 2, (0.5 µg/ml) over night at 4°C. The blots were washed 4x 5 min in TBS-Tween20 and horse radish peroxidase-conjugated goat anti-rabbit IgG antibody (DAKO) diluted 1:3000 in blocking buffer was applied and incubated for 1 h at RT. After washing as above, the detection was performed with TMBM substrate (MOSS inc.) and 15 min development. The blots were finally rinsed in dH20, and scanned with a table top scanner. Band intensities were translated to relative intensities using the publicly available image analysis tool ImageJ (http://rsbweb.nih.gov/ij/).

### b) Results

To verify that the antibodies, obtained as described in Example 2, target the correct protein, Western blots with plasma samples were performed with these antibodies as well as a mouse monoclonal anti-fibulin 1 antibody (sc-25281, Santa Cruz Biotechnology). Four samples from the GN category showing high signal intensity profiles for fibulin 1 as well as their respective matched controls were chosen for Western blot detection. All antibodies detected the same target protein of about 70-80 kDa in plasma, which corresponds to a predicted size for fibulin 1 of 77 kDa, (according to Uniprot P23142). As shown in figure 4A, using the polyclonal anti-fibulin 1 antibody denoted HPA1, the intensity profile determined in the Western blot bands were congruent with those obtained from the antibody array analysis (figure 4B). This confirms that the difference between cases and controls in the discovery screening was associated to relative abundance of fibulin 1.

### 6. Glomerulonephritis, cohort II

### a) Materials and methods

Plasma samples were collected at the Charite Hospital in Berlin (Germany) from 26 glomerulonephritis (GN) patients and 11 healthy controls. The age of the GN patients ranged from 21 to 79 years, and the age of the healthy controls ranged from 22 to 87 years. Seven of the patients were female and three of the healthy controls were female. Serum creatinine levels ranged from 8 to 75 µg/ml, and BUN levels ranged from 84 to 1350 µg/ml for the GN patients. All samples were stored at -80°C until use, and then analyzed as described in Example 3.

### b) Results

Co-profiling of a subset of the previously analyzed samples (8 cases + 8 controls) from cohort I (see Example 4) and the current cohort (26 cases + 11 controls) confirmed fibulin 1 as a marker discriminating GN cases from controls in each cohort separately and in a statistically significant manner for the polyclonal anti-fibulin 1 antibodies, obtained as described in Example 2 (figure 5).

### 7. Fibulin 1 in four types of kidney disorders

### a) Materials and methods

Plasma samples were obtained from Binding Site Inc. Lithium heparin plasma samples were collected from 20 consenting individuals representing four patient groups with impaired renal function to various degrees. The disease groups included patients representing renal glomerulonephritis (GN, cohort I described in Example 4), diabetic nephropaty (DN) and obstructive uropathy (OU) as well as patients experiencing renal dysfunction or damage due to abuse of analgesic substances (AA). All patients were diagnosed based on ultrasound and/or X-ray imaging, supported with a CT scan for a smaller portion of the patients. In addition, plasma samples were collected from 20 healthy individuals matched in age, gender and body mass index. All samples were stored at -80°C until use, and then analyzed as described in Example 3, using the polyclonal antibodies obtained as described in Example 2.

### b) Results

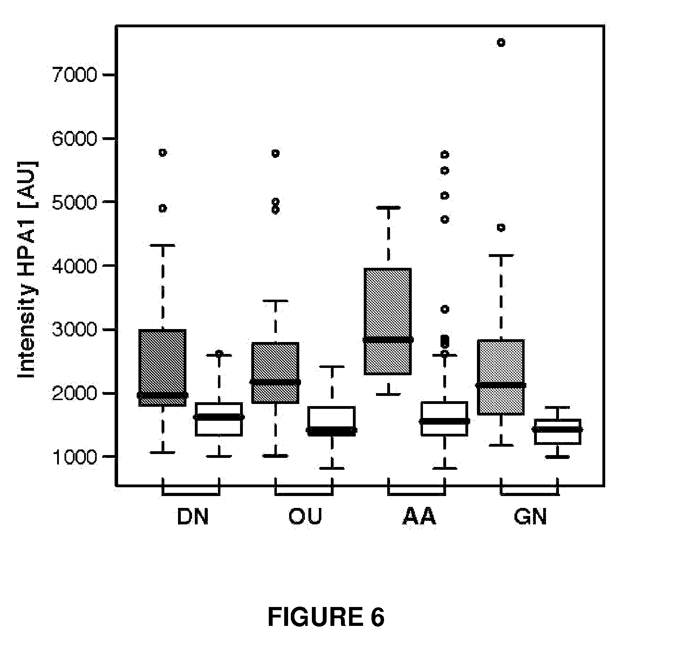

Fibulin 1 was found to be able to separate all kidney disease categories investigated, apart from the analgesic abuse (AA) category, from their controls (p-values < 0.05 considered significant) using two of the polyclonal anti-fibulin 1 antibodies (denoted HPA1 and HPA2). For the AA category there was a trend toward separating the two groups (healthy and diseased), but for an unknown reason there was a large difference in fibulin 1 protein expression in the control group for this category. This could possibly explain the lack of significance in separating case and control for the AA category. As shown in Figure 6, there was a higher level of fibulin 1 protein in plasma from patients diagnosed with kidney disorders (grey boxes) than in plasma from healthy individuals (white boxes). Mean values and 95% confidence intervals of the ratios between the sample group medians are presented with corresponding p-values in Table 2, the p-values corresponding to the results shown in Figure 6 are listed under *Exp 1.*

**Table 2. Discriminatory capacity in the form of p-values of fibulin 1 in case control comparisons across four types of kidney disorders.**

| | **HPA1** | | **HPA2** | | **HPA3** | |
|---|---|---|---|---|---|---|
| **Disorder** | *Exp 1* | *Exp 2* | *Exp 1* | *Exp 2* | *Exp 1* | *Exp 2* |
| Diabetic nephropathy | 0.004 | 0.007 | 0.003 | 0.006 | 0.1 | 0.1 |
| Obstructive uropathy | 0.0002 | 0.0005 | 0.003 | 0.0008 | 0.02 | 0.01 |
| Analgesic abuse | 0.05 | 0.09 | 0.09 | 0.1 | 0.01 | 0.2 |
| Glomerulonephritis | 0.0003 | 0.0003 | 0.00001 | 0.000005 | 0.004 | 0.002 |

### 8. Protein detection using the sandwich immunoassay

### a) Materials and methods

Plasma samples from glomerulonephritis (GN) patients in cohort I (Example 4) were treated similarly to the direct labeling procedure as described in Example 3, but utilized without biotinylation. Samples were thawed at room temperature, diluted 1/10 in PBS followed by 1/50 in PVXCas buffer and heat treated for 30 min at 56°C. Beads coupled with anti-fibulin 1 antibodies obtained as described in Example 2, were incubated overnight together with the samples under permanent shaking. The beads were then washed 3x 100 µl PBST. A mouse monoclonal anti-fibulin 1 antibody (sc-25281, Santa Cruz Biotechnology) was biotinylated using a 50x molar excess of PEO4-biotin (Pierce) over antibody, and was diluted as detection reagent to 0.4 µg/ml. After 60 min incubation, the beads were washed as described above, and Phycoerythrin-modified streptavidin 0.6 µg/ml in PBST was added and incubated for 20 min with the beads. Following a final washing step the beads were measured in the LX200 instrumentation in 100 µl PBST.

### b) Results

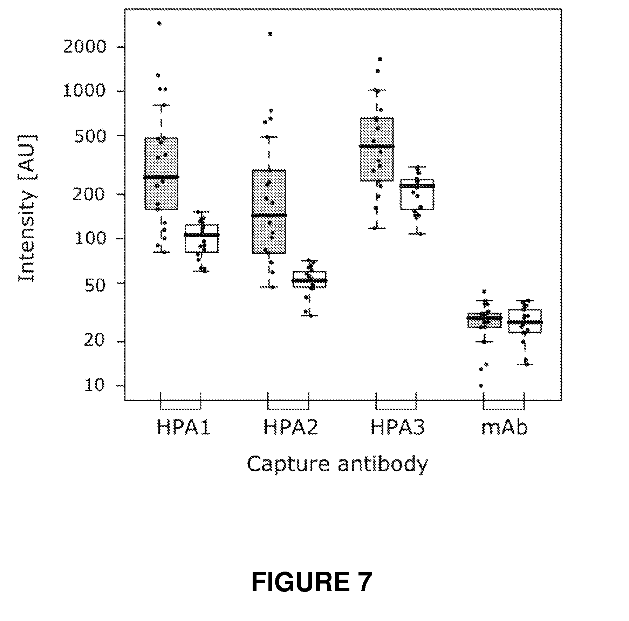

Samples from patients diagnosed with glomerulonephritis (GN) as well as samples from healthy individuals were analyzed using the sandwich immunoassay. The anti-fibulin 1 polyclonal antibodies HPA 1-3 were used as capture antibodies and mouse monoclonal anti-fibulin 1 antibody (sc-25281, Santa Cruz Biotechnology), was used as detection reagent. Again, patients with glomerulonephritis (GN) were shown to have higher levels of the fibulin 1 protein than the healthy individuals (Figure 7). The discrepancy between GN disease and control patients was much larger than in the normal plasma profiling assay, with an average sick/healthy ratio up to 20-fold for HPA2.

In conclusion, the results from this sandwich assay, which is an assay frequently applied in a clinical setting, shows that the fibulin 1 protein is a biomarker indicating renal damage that can be detected using established routine assays.

### 9. Detection of fibulin 1 in urine

### a) Materials and methods

Urine samples were collected from diabetic nephropaty (DN) patients, diagnosed as described in Example 7, and handled essentially as described in Example 3.

### b) Results

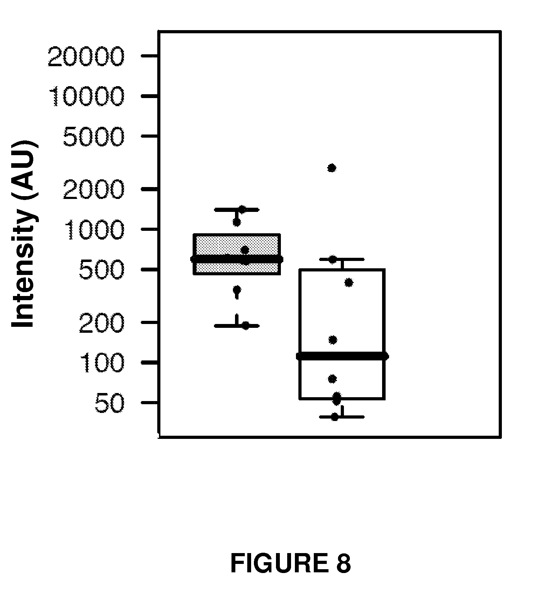

There was a trend toward a higher level of fibulin 1 protein in urine samples from patients with diabetic nephropathy (DN) than in urine from the healthy controls as can be seen in Figure 8. However, the number of samples available for analysis were too few for a significant difference between the groups.

## Claims

1. A method of determining whether a subject belongs to a first or a second group of subjects, wherein the risk of having or developing of a renal impairment is higher in the first group than in the second group, comprising the steps of:
a) measuring an amount of fibulin 1 in a sample from the subject to obtain a sample value;
b) comparing the sample value to a reference value; and
if the sample value is higher than the reference value,
c1) concluding that the subject belongs to the first group; and
if the sample value is lower than the previous sample value,
c2) concluding that the subject belongs to the second group.

2. A method according to claim 1, wherein the subject undergoes a clinical trial of a treatment.

3. A method of monitoring a renal condition in a subject having a renal disorder, comprising the steps of:
a) measuring an amount of fibulin 1 in a sample from the subject to obtain a sample value;
b) comparing the sample value to a previous sample value obtained from an earlier sample taken from the subject; and
if the sample value is higher than the previous sample value,
c1) concluding that the renal condition has worsened since the earlier
sample was taken; and/or
if the sample value is lower than the previous sample value,
c2) concluding that the renal condition has improved since the earlier
sample was taken.

4. A method according to claim 3, wherein the subject participates in a clinical trial of a potential treatment of the renal disorder and the earlier sample was taken earlier in the clinical trial process or before the clinical trial was commenced.

5. A method of disqualifying a subject from a clinical trail of a treatment, comprising the steps of:
a) measuring an amount of fibulin 1 in a sample from the subject to obtain a sample value;
b) comparing the sample value to a reference value; and
if the sample value is higher than the reference value,
c) disqualifying the subject from the clinical trial.

6. A method according to any one of the preceding claims, wherein the sample is an optionally modified sample derived from urine or blood, such as an optionally diluted serum or plasma sample.

7. A method according to any one of the preceding claims, wherein step a) comprises contacting the sample with an affinity ligand capable of selective interaction with fibulin 1, such as an affinity ligand is capable of selective interaction with a peptide whose amino acid sequence consists of SEQ ID NO:1 or SEQ ID NO:2.

8. A kit comprising:
a) a quantifiable affinity ligand capable of selective interaction with a fibulin 1 protein;
b) reagents necessary for quantifying the amount of the quantifiable affinity ligand of a);
a') a quantifiable affinity ligand capable of selective interaction with GC, SLC13A3, GDA, MACF1, MAPK3, RBP4, SFXN1 or BBOX1; and
b') reagents necessary for quantifying the amount of the quantifiable affinity ligand of a'),
wherein the reagents of b) and b') may be the same or different.

9. A kit comprising:
c) a quantifiable affinity ligand capable of selective interaction with a fibulin 1 protein;
d) reagents necessary for quantifying the amount of the quantifiable affinity ligand of a);
a') a quantifiable affinity ligand capable of selective interaction with Serum Creatinine, KIM-1, Cystatin C, L-FABP, alpha-GST, pi-GST, Urinary Collagen IV, Alpha-1-Microglobulin, Beta-2-Microglobulin, Calbindin, Clusterin, Connective Tissue Growth Factor, Glutathione S-Transferase alpha, Urinary albumin, Microalbumin, NGAL, Osteopontin, Tamm-Horsfall Urinary Glycoprotein, Tissue Inhibitor of Metalloproteinases 1, TFF3 or VEGF; and
b') reagents necessary for quantifying the amount of the quantifiable affinity ligand of a'),
wherein the reagents of b) and b') may be the same or different.

10. A product comprising an affinity ligand capable of selective interaction with fibulin 1, which affinity ligand is immobilized onto a solid support.

11. Kit comprising:
a) a first affinity ligand capable of selective interaction with a fibulin 1 protein;
b) a quantifiable second affinity ligand capable of interaction with the fibulin 1 protein when bound to the first affinity ligand; and optionally
c) a solid support onto which the first affinity ligand is immobilized or which is prepared for immobilization of the first affinity ligand.

12. Kit according to claim 11, wherein the first affinity ligand interacts with a first fibulin 1 amino acid sequence and the quantifiable second affinity ligand interacts with a second fibulin 1 amino acid sequence, which is different from the first amino acid sequence.

13. Use *ex vivo* of fibulin 1 as a diagnostic marker for a renal impairment.

14. Use *ex vivo* of fibulin 1 as a diagnostic marker for a renal impairment in a clinical trial of a treatment.

15. Use *ex vivo* of an affinity ligand capable of selective interaction with fibulin 1 as a diagnostic agent for a renal impairment.
